# EUROPÄISCHE PATENTANMELDUNG

(11) **EP 4 763 125 A1**
(43) Veröffentlichungstag der Anmeldung: **24.06.2026**
(21) Anmeldenummer: 25218572.3
(22) Anmeldetag: 26.11.2025
(51) Int. Cl.: A61B 34/30, A61B 34/32, A61B 34/37, A61B 34/00

(54) **SYSTEM ZUR OPTIMIERUNG EINES KONTROLLPUNKTS EINES ROBOTISCH ANSTEUERBAREN INSTRUMENTS**

(30) Priorität: 19.12.2024 DE 102024138989
(71) Anmelder: Karl Storz SE & Co. KG, 78532 Tuttlingen (DE)
(72) Erfinder: Sharma, Shashank, 78532 Tuttlingen (DE); Kana, Sreekanth, 78532 Tuttlingen (DE)

(57) **Zusammenfassung**

Es werden ein System (100) und ein Verfahren zur dynamischen Optimierung eines Kontrollpunkts (K-i) mindestens eines robotisch ansteuerbaren Instruments (P-i), bereitgestellt. Das System (100) umfasst: eine Erfassungseinrichtung (10), welche dazu eingerichtet ist, Informationen über eine Position und einen Kontrollpunkt (K-i) des mindestens einen robotisch ansteuerbaren Instruments (P-i) fortlaufend zu erfassen; eine Ermittlungseinrichtung (20), welche dazu eingerichtet ist, auf Basis der Informationen über die Position und den Kontrollpunkt (K-i) des mindestens einen robotisch ansteuerbaren Instruments (P-i), mindestens einen optimierten Kontrollpunkt (K-i) zu ermitteln; und eine Steuerungseinrichtung (30), welche dazu eingerichtet ist, Bewegungsbefehle (B0) an einen Roboter (R1), der das mindestens eine robotisch ansteuerbare Instrument (P-i) steuert, zu übertragen, wobei die Bewegungsbefehle (B0) dazu ausgelegt sind, den Roboter (R1) anzuweisen, das mindestens eine robotisch ansteuerbare Instrument (P-i) von einem des mindestens einen optimierten Kontrollpunkts (K-i) aus zu kontrollieren.

## Beschreibung

### Technisches Gebiet der Erfindung

Die vorliegende Erfindung betrifft ein System und ein Verfahren zur dynamischen Optimierung eines Kontrollpunkts mindestens eines robotisch ansteuerbaren Instruments, insbesondere im medizinischen Bereich. Die vorliegende Erfindung betrifft ferner ein Master-Slave-System zur Durchführung medizinischer Prozeduren.

### Hintergrund der Erfindung

Bei medizinischen Verfahren und Eingriffen werden die Chirurgen/Operateure in der Regel durch teilweise oder vollständig robotisierte Systeme unterstützt. Dies gilt insbesondere für die robotergestützte Chirurgie, bei der ein oder mehrere Roboter für die Handhabung verschiedener medizinischer Instrumente und Geräte eingesetzt werden, die von einem Chirurgen/Operateur überwacht, programmiert oder ferngesteuert werden. Der Einsatz von Roboterkomponenten im medizinischen Bereich führt zu einer Erhöhung der Genauigkeit und erleichtert somit die Arbeit des Chirurgen.

Bei der Fernsteuerung von Robotersystemen, insbesondere bei Master-Slave-Systemen, wird versucht, die Bewegungsbefehle des Chirurgen so ergonomisch wie möglich zu gestalten. Die Beweglichkeit eines Roboterarms und die Wahl des Kontrollpunkts, von dem aus der Roboter das medizinische Instrument oder Gerät hält, sind hier wichtige Parameter.

Derzeit arbeiten die meisten Robotersysteme in der robotergestützten Chirurgie mit einem Kontrollpunkt für ein Instrument, der vor einer medizinischen Prozedur festgelegt wird.

Im Laufe einer medizinischen Prozedur sind jedoch bestimmte Bewegungen eines Instruments von dem vorgegebenen Kontrollpunkt aus nicht ergonomisch. Die daraus resultierenden Bewegungen, die der Roboter mit dem Instrument ausführt, können für den Chirurgen fremd oder unüblich sein. Dies könnte dazu führen, dass die Fernsteuerung des Roboters als nicht intuitiv empfunden wird. Dies stellt manchmal eine Einschränkung dar und erschwert die Arbeit des Chirurgen.

Es ist daher wichtig, Systeme und Verfahren bereitzustellen, welche die Fernsteuerung von Robotern in Master-Slave-Systemen für den Chirurgen intuitiv und ergonomisch gestalten.

### Zusammenfassung der Erfindung

Es ist somit eine Aufgabe der vorliegenden Erfindung, ein System und ein Verfahren bereitzustellen, die eine verbesserte Fernsteuerung eines Roboters, der mindestens ein Instrument kontrolliert, ermöglichen.

Diese Aufgabe wird durch die Gegenstände der unabhängigen Patentansprüche der vorliegenden Erfindung gelöst.

Gemäß einem ersten Aspekt wird demnach ein System zur dynamischen Optimierung eines Kontrollpunkts mindestens eines robotisch ansteuerbaren Instruments bereitgestellt, umfassend: eine Erfassungseinrichtung, welche dazu eingerichtet ist, Informationen über eine Position und einen Kontrollpunkt des mindestens einen robotisch ansteuerbaren Instruments fortlaufend zu erfassen; eine Ermittlungseinrichtung, welche dazu eingerichtet ist, auf Basis der Informationen über die Position und den Kontrollpunkt des mindestens einen robotisch ansteuerbaren Instruments, mindestens einen optimierten Kontrollpunkt zu ermitteln; und eine Steuerungseinrichtung, welche dazu eingerichtet ist, Bewegungsbefehle an einen Roboter, der das mindestens eine robotisch ansteuerbare Instrument steuert, zu übertragen, wobei die Bewegungsbefehle dazu ausgelegt sind, den Roboter anzuweisen, das mindestens eine robotisch ansteuerbare Instrument von einem des mindestens einen optimierten Kontrollpunkts zu kontrollieren, insbesondere zu bewegen.

Eine grundlegende Idee der vorliegenden Erfindung besteht darin, den Kontrollpunkt eines robotergesteuerten Instruments dynamisch (d.h. fortlaufend oder kontinuierlich) während einer Prozedur anpassen zu können. Das erfindungsgemäße System ist dazu ausgelegt, neue Kontrollpunkte basierend auf mindestens einem bestehenden Kontrollpunkt und einer Position (oder Orientierung) des Instruments zu ermitteln, wobei die neuen Kontrollpunkte während der Prozedur eingesetzt werden können.

Während einer medizinischen Prozedur mit handgeführten Instrumenten wird die Haltung, in der ein Chirurg seine Instrumente hält, ständig verändert. Damit versucht der Chirurg, den optimalen Halt des Instruments zu finden, der unter anderem von der auszuführenden Tätigkeit in den verschiedenen Phasen der Operation abhängt. Bei robotergestützten Eingriffen hält der Chirurg das Instrument nicht selbst, sondern mittels eines Roboterarms, der von dem Chirurgen über eine Bedieneinheit gesteuert wird. Die Bedieneinheit kann den Roboter (und damit das Instrument) über einen Joystick und/oder durch Sprachbefehle steuern. Chirurgen haben ihre Gewohnheiten und Fähigkeiten durch Operationen mit handgehaltenen Instrumenten entwickelt. Bei den meisten herkömmlichen Robotersystemen ist es schwierig, diese Gewohnheiten beizubehalten bzw. die Fähigkeiten voll zu entfalten, da die Steuerung des Instruments nicht direkt durch den Chirurgen, sondern indirekt über den Roboter erfolgt.

Ein Vorteil der vorliegenden Erfindung besteht darin, dass mit dem erfindungsgemäßen System der Kontrollpunkt eines Instruments nicht mehr statisch ist, sondern dynamisch verändert werden kann, insbesondere automatisch. Somit kommen die Bewegungen eines Roboters in Master-Slave-Systemen denen eines Chirurgen näher. Die Auswahl der optimierten Kontrollpunkte erfolgt automatisch.

Auf diese Weise ahmt das System die Bewegungen eines Chirurgen auf realistischere Weise nach, sodass der Chirurg die durch den Roboter auszuführenden oder ausgeführten Bewegungen wie seine eigenen empfinden kann. Die Möglichkeit, den Kontrollpunkt während einer medizinischen Prozedur anzupassen, gibt dem Chirurgen Flexibilität bei der Interaktion mit dem Instrument und ermöglicht es ihm, die effektivste Kontrollstrategie für jede spezifische chirurgische Aufgabe zu wählen. Beispielsweise ist beim Nähen die präzise Steuerung der Instrumentenspitze für die Genauigkeit entscheidend. Umgekehrt kann die Festlegung des Kontrollpunkts am Handgelenk des Instruments in Szenarien wie der Vorbereitung komplexer Operationen von Vorteil sein, in denen die Feineinstellung der Ausrichtung des Handgelenks sicherstellt, dass das Instrument optimal ausgerichtet ist, bevor man sich auf die Bewegung der Spitze konzentriert.

Vorteilhaft kann das System des ersten Aspekts der Erfindung für alle Arten von robotergestützten Prozeduren (einschließlich medizinischen Prozeduren) verwendet werden, und für alle Arten von Instrumenten.

Unter einem robotisch ansteuerbaren Instrument ist hierin ein Instrument oder ein Gerät zu verstehen, das durch den Einsatz eines Roboters betrieben oder bedient werden kann.

Der Roboter kann ein Roboter mit einem Mehrgelenkroboterarm sein, der an seinem distalen Ende einen Instrumentenhalter besitzt.

Ein Kontrollpunkt ist eine Stelle an einem Instrument oder an einem Gerät, die als Referenzpunkt bzw. Startpunkt für die Übertragung der Bewegung des Chirurgen von der Bedienvorrichtung auf das Instrument dient. Ein Kontrollpunkt kann von einem Roboter als Haltepunkt (d.h. als Fixpunkt) verwendet werden kann, um das Instrument von dort aus zu steuern, insbesondere zu bewegen. Mit anderen Worten, ein Kontrollpunkt ist ein Punkt, auf den sich die Bewegungssteuerung (Handbewegungen des Chirurgen, die vom Roboter übertragen werden) konzentriert (und in manchen Fällen beschränkt).

Informationen über eine Position eines Instruments umfassen in der Regel zumindest Informationen über die Lage zumindest einiger Grenzen des Instruments und/oder deren Orientierung. Beispielsweise können (etwa durch eine Bildregistrierung) den Objektgrenzen Koordinaten eines Bezugssystems zugeordnet werden. Unter einer Bestimmung der Position eines Instruments ist insbesondere eine solche Bestimmung von Ortskoordinaten zu verstehen.

Bewegungsbefehle enthalten Informationen für den Roboter (vorzugsweise in Maschinensprache), damit der Roboter den Haltepunkt eines Instruments (von einem bestehenden Kontrollpunkt zu einem anderen Kontrollpunkt hin) ändern kann.

Obwohl hier, im Vorstehenden und im Folgenden einige Funktionen als von "Einrichtungen" oder "Schnittstellen" ausgeführt beschrieben werden, versteht es sich, dass dies nicht unbedingt bedeutet, dass solche Einrichtungen, oder Schnittstellen als voneinander getrennte Vorrichtungen bereitgestellt werden. In Fällen, in denen eine oder mehrere Einrichtungen oder Schnittstellen ganz oder teilweise als Software bereitgestellt werden, können die Einrichtungen oder Schnittstellen durch Programmcodeabschnitte oder Programmcodeschnipsel implementiert werden, die sich voneinander unterscheiden, aber auch miteinander verwoben sein können.

In ähnlicher Weise können in dem Fall, in dem ein oder mehrere Einrichtungen oder Schnittstellen als Hardware bereitgestellt werden, die Funktionen einer oder mehrerer Einrichtungen oder Schnittstellen von ein und derselben Hardwarekomponente bereitgestellt werden, oder die Funktionen einer Einrichtung oder einer Schnittstelle, oder die Funktionen mehrerer Einrichtungen oder Schnittstellen können auf mehrere Hardwarekomponenten verteilt sein, die nicht notwendigerweise den Einrichtungen oder Schnittstellen eins zu eins entsprechen müssen. Daher ist jede Vorrichtung, jedes System, jedes Verfahren usw., das alle Merkmale und Funktionen aufweist, die einer bestimmten Einrichtung und/oder einer bestimmten Schnittstelle zugeschrieben werden, so zu verstehen, dass sie bzw. es die Einrichtung und/oder die Schnittstelle ausbildet, umfasst, oder implementiert.

Insbesondere besteht die Möglichkeit, dass einige oder alle Einrichtungen und Schnittstelle durch ausführbare Programmcode implementiert werden.

Insbesondere die Erfassungseinrichtung und/oder die Ermittlungseinrichtung und/oder die Steuerungseinrichtung kann als jede Vorrichtung oder jedes Mittel zum Rechnen realisiert werden, insbesondere zum Ausführen einer Software, einer App oder eines Algorithmus. Beispielsweise kann die Erfassungseinrichtung und/oder die Ermittlungseinrichtung und/oder die Steuerungseinrichtung mindestens einen Prozessor, wie etwa mindestens einen Zentralprozessor, CPU und/oder mindestens einen Grafikprozessor, GPU, und/oder mindestens ein feldprogrammierbares Gate-Array, FPGA, und/oder mindestens einen anwendungsspezifischen integrierten Schaltkreis, ASIC, und/oder eine beliebige Kombination der Vorstehenden umfassen. Die Erfassungseinrichtung und/oder die Ermittlungseinrichtung und/oder die Steuerungseinrichtung kann ferner einen Arbeitsspeicher aufweisen, der mit dem mindestens einen Prozessor operativ verbunden ist, und/oder einen nicht-flüchtigen Speicher, der mit dem mindestens einen Prozessor und/oder dem Arbeitsspeicher operativ verbunden ist. Die Erfassungseinrichtung und/oder die Ermittlungseinrichtung und/oder die Steuerungseinrichtung kann teilweise und/oder vollständig in einer lokalen Vorrichtung (wie zum Beispiel in einer Bedieneinheit oder in einer Bedienkonsole eines roboterassistierten Chirurgiesystems) und/oder teilweise und/oder vollständig in einem entfernten System, wie z. B. durch eine Cloud-Computing-Plattform, implementiert sein. Die Ermittlungseinrichtung kann insbesondere mit Maschinenlernen-Modellen ausgestattet werden.

Die Erfassungseinrichtung kann dazu eingerichtet sein, die Position und den Kontrollpunkt des Instruments durch die Verarbeitung von Bilddaten einer oder mehrerer Bildgebungseinrichtungen (z.B. des Bildstroms eines Endoskops) zu erfassen, insbesondere in Echtzeit, oder alternativ auch von einem Bildablage- und Kommunikationssystem (PACS, von engl. "picture archiving and communications system"), wobei auch letzteres in Echtzeit erfolgen kann, also insbesondere sobald die Bilddaten in dem PACS eingehen.

Weitere Vorteile der Erfindung werden im Folgenden anhand der Gegenstände der Unteransprüche und insbesondere anhand der Beschreibung der Figuren erläutert.

Gemäß einigen bevorzugten Ausführungsformen, Varianten oder Verfeinerungen von Ausführungsformen ist das robotisch ansteuerbare Instrument ein medizinisches Instrument, insbesondere zur Anwendung bei minimalinvasiven endoskopischen Prozeduren. Obwohl die Prinzipien der Erfindung auf beliebige Master-Slave-Systeme anwendbar sind, werden Anwendungen im medizinischen Bereich, insbesondere robotergestützte minimalinvasive Verfahren, bevorzugt. Beispiele für robotergesteuerte Instrumente sind in diesem Zusammenhang Pinzetten, Zangen, Nadelhalter oder Klammernahtgeräte.

Gemäß einigen bevorzugten Ausführungsformen, Varianten oder Verfeinerungen von Ausführungsformen ist die Ermittlungseinrichtung ferner dazu eingerichtet, einen optimierten Kontrollbereich zu ermitteln. Statt nur einen oder mehrere Kontrollpunkte zu ermitteln, kann die Ermittlungseinrichtung einen ganzen Bereich anbieten. Dies dient dazu, die Auswahl der optimierten Kontrollpunkte flexibler zu gestalten, um die Gewohnheiten der verschiedenen Operateure besser berücksichtigen zu können. Der optimierte Kontrollbereich kann beispielsweise das Maulteil einer Zange bzw. Fasszange sein. Bei diesen Ausführungsformen kann ein Operateur, der das Maulteil z.B. von einem maximal distalen Kontrollpunkt aus kontrollieren möchte, seinen persönlichen optimalen Kontrollpunkt auswählen.

Gemäß einigen bevorzugten Ausführungsformen, Varianten oder Verfeinerungen von Ausführungsformen umfasst das erfindungsgemäße System ferner eine Benutzerschnittstelle, die dazu ausgelegt ist, eine Mehrzahl von Kontrollpunkten anzuzeigen und einen daraufhin von einem Benutzer (oder Operateur) ausgewählten Kontrollpunkt aus der Mehrzahl von Kontrollpunkten als den optimierten Kontrollpunkt an die Steuerungseinrichtung zu übertragen. In einigen Ausführungsformen der Erfindung ist es vorgesehen, dass die Ermittlungseinrichtung mehr als einen optimierten Kontrollpunkt ermittelt.

Die Benutzerschnittstelle ermöglicht es dem Operateur beispielsweise, einen neuen Kontrollpunkt aus der Mehrzahl von Kontrollpunkten auszuwählen. Die Benutzerschnittstelle kann vorzugsweise dazu ausgelegt sein, den aktuellen (bestehenden) Kontrollpunkt zusammen mit dem mindestens einen ermittelten Kontrollpunkt anzuzeigen. Die Kontrollpunkte können insbesondere farbcodiert, zahlencodiert, buchstabencodiert etc. dargestellt werden, wobei die Codierung einer nach einer Rangfolge geordneten Empfehlungsliste der Ermittlungseinrichtung entspricht.

Gemäß einigen bevorzugten Ausführungsformen, Varianten oder Verfeinerungen von Ausführungsformen umfasst die Benutzerschnittstelle ein Display und einen Regler, wobei das Display dazu ausgelegt ist, eine Mehrzahl von Kontrollpunkten anzuzeigen, und wobei der Regler so ausgelegt ist, dass ein Benutzer (oder Operateur) damit einen gewünschten Kontrollpunkt aus der Mehrzahl von Kontrollpunkten auswählen kann. Das Display kann so ausgelegt sein, dass es die Mehrzahl von Kontrollpunkten einem Bild des Instruments überlagert. Der Operateur kann dann mit dem Regler durch das Bild des Instruments navigieren, beispielsweise mit einem Cursor, und einen optimierten Kontrollpunkt mit dem Cursor graphisch auswählen.

Wie oben erläutert, kann das erfindungsgemäße System auch optimierte Kontrollbereiche graphisch darstellen. Kontrollpunkte z.B. innerhalb des Maulteils (als ein Beispiel für einen Kontrollbereich) können mit Hilfe des Reglers ausgewählt werden. Bei medizinischen Prozeduren, bei denen eine Mehrzahl von Instrumenten von einem Roboter gesteuert werden, kann die Benutzerschnittstelle auch einen Mechanismus enthalten, mithilfe dessen der Operateur zunächst ein Instrument aus der Mehrzahl von Instrumenten auswählen kann. Dies kann beispielsweise in einem Menü auf dem Display angezeigt werden.

Gemäß einigen bevorzugten Ausführungsformen, Varianten oder Verfeinerungen von Ausführungsformen ist die Ermittlungseinrichtung ferner dazu eingerichtet, den optimierten Kontrollpunkt aus einer Liste von vorbestimmten Kontrollpunkten auszuwählen, wobei die Liste von vorbestimmten Kontrollpunkten abhängig von dem robotisch ansteuerbaren Instrument ist. Bei diesen Ausführungsformen der Erfindung sind die Kontrollpunkte somit auf die vorgegebene Liste beschränkt. Dies spart Rechenressourcen. Beispielsweise könnte die Liste von Kontrollpunkten für eine Fasszange einen Kontrollpunkt an einem Gelenk, einen Kontrollpunkt an einem Maulteil und einem Kontrollpunkt an einer Spitze umfassen. Die Ermittlungseinrichtung kann somit im Verlauf einer medizinischen Prozedur bestimmen, welcher dieser drei Kontrollpunkte der optimierte Kontrollpunkt ist.

Die Anzahl und Position der vorgegebenen Kontrollpunkte hängt von dem Instrument ab. Ein komplexes Instrument kann beispielsweise mehrere vorgegebene Kontrollpunkte besitzen.

Gemäß einigen bevorzugten Ausführungsformen, Varianten oder Verfeinerungen von Ausführungsformen umfasst die Ermittlungseinrichtung ferner eine künstliche-Intelligenz-Entität, die mit einem oder mehreren Modellen des maschinellen Lernens (oder Maschinenlernen-Modellen) ausgestattet ist, und welche dazu trainiert und ausgebildet ist, einen optimierten Kontrollpunkt automatisch zu wählen. Die Algorithmen der Ermittlungseinrichtung können zumindest teilweise mit Methoden der künstlichen Intelligenz unterstützt werden, beispielsweise von diesen gespeist werden oder Eingabedaten für diese bereitstellen.

Das oder die Maschinenlernen-Modelle können auch Modelle des tiefen Lernens umfassen, die ein oder mehrere künstliche neuronale Netze implementieren. Unter künstlichen neuronalen Netzen sind hier Modelle mit einer beliebigen Architektur und einer beliebigen Anzahl von Zwischenschichten zu verstehen, wie etwa Convolutional Neural Networks (CNNs). Die Ermittlung optimierter Kontrollpunkte kann mit einem typischen künstlichen neuronalen Netzwerk aus dem Stand der Technik erfolgen, beispielsweise mit einem ResNet50, das mit Daten aus medizinischen Prozeduren trainiert wurde, bei denen die Instrumente von Chirurgen behandelt werden.

Gemäß einigen bevorzugten Ausführungsformen, Varianten oder Verfeinerungen von Ausführungsformen ist die künstliche-Intelligenz-Entität dazu ausgelegt, den optimierten Kontrollpunkt basierend auf Informationen über mindestens ein zu verwendendes robotisch ansteuerbares Instrument und/oder über einen Benutzer (z.B. einen Operateur) und/oder über eine durchzuführende Prozedur automatisch auszuwählen. Die optimalen Kontrollpunkte werden vorzugsweise mit so vielen relevanten Informationen wie möglich ermittelt. Die optimierten Kontrollpunkte können somit unter Berücksichtigung des Instruments und der Art der Prozedur bestimmt werden. Die Kontrollpunkte können auch für jeden Operateur individuell angepasst werden. Verschiedene Operateur handhaben die Instrumente nämlich mitunter nach unterschiedlichen Präferenzen. Die künstliche-Intelligenz-Entität kann die Kontrollpunkte an diese Präferenzen eines Operateurs anpassen.

Gemäß einigen bevorzugten Ausführungsformen, Varianten oder Verfeinerungen von Ausführungsformen können die ermittelten Kontrollpunkte für einen Operateur in einer Datenbank gespeichert werden. Auf diese Weise kann die Ermittlungseinrichtung die von einem Operateur bevorzugten Kontrollpunkte abrufen und dementsprechend die ermittelten Kontrollpunkte unter Berücksichtigung der Präferenzen des Chirurgen bestimmen.

Gemäß einigen bevorzugten Ausführungsformen, Varianten oder Verfeinerungen von Ausführungsformen ist die künstliche-Intelligenz-Entität dazu ausgelegt, den optimierten Kontrollpunkt mit Hilfe von Informationen mindestens eines optischen Sensors zu bestimmen. Ferner ist der mindestens eine optische Sensor (der Teil des Systems oder separat davon ausgebildet sein kann) dazu eingerichtet, Bilder von zumindest dem robotisch ansteuerbaren Instrument und dem Kontrollpunkt zu erfassen. Bilddaten von einem optischen Sensor (z. B. der Kamera eines Endoskops) können verwendet werden, um die Position der Kontrollpunkte zu verfeinern. Bei diesen Ausführungsformen ist die künstliche-Intelligenz-Entität, KIE, vorzugsweise mit einer Bildverarbeitungssoftware ausgestattet, um eine Erkennung des Instruments zu ermöglichen oder zu präzisieren.

Beispielsweise kann mittels der KIE automatisch ein Gewebe in der Nähe des Instruments ermittelt werden, und basierend auf der Position des Gewebes automatisch der optimierte Kontrollpunkt ermittelt werden. In einigen Ausführungsformen kann automatisch mittels der KIE auch eine Eigenschaft des Gewebes, beispielsweise ein Gewebetyp oder dergleichen, ermittelt werden, und das Ermitteln des optimierten Kontrollpunkts unter anderem darauf basierend erfolgen.

Gemäß einigen bevorzugten Ausführungsformen, Varianten oder Verfeinerungen von Ausführungsformen ist die künstliche-Intelligenz-Entität ferner dazu ausgebildet, den optimierten Kontrollpunkt gemäß einer Operationsplanung (OP-Planung) zu ermitteln. Unter einer OP-Planung ist hier eine Zusammenstellung von Informationen zu verstehen, welche den Ablauf der medizinischen Prozedur beschreiben. Die künstliche-Intelligenz-Entität kann beispielsweise mit Methoden der generativen künstlichen Intelligenz ausgestattet sein, so dass sie basierend auf den Anweisungen der OP-Planung die optimierten Kontrollpunkte bestimmen kann. In diesen Ausführungsformen kann die Ermittlungseinrichtung ferner dazu ausgebildet sein, eine solche OP-Planung beispielsweise von einer lokalen Benutzerschnittstelle oder von einer zentralen Recheneinrichtung zu empfangen.

Gemäß einigen bevorzugten Ausführungsformen, Varianten oder Verfeinerungen von Ausführungsformen umfasst das erfindungsgemäße System ferner mindestens einen Kraftsensor, der dazu ausgelegt ist, Informationen über die von dem robotisch ansteuerbaren Instrument ausgeübte oder ausübbare Kraft zu erfassen und die erfassten Informationen an die Ermittlungseinrichtung zu übertragen. Informationen über eine ausgeübte Kraft sind beispielsweise relevant, um einen besseren Kontrollpunkt zu suchen, z.B. einen Kontrollpunkt von dem aus Kräfte effizienter übertragen werden können. Das Ermitteln des mindestens einen optimierten Kontrollpunkts kann somit optional unter anderem basierend auf der Information über die ausgeübte oder ausübbare Kraft basieren; beispielsweise kann sie derart erfolgen, dass die ausübbare Kraft maximiert wird, oder derart, dass die ausübbare Kraft besonders präzise dosierbar ist.

Gemäß einem zweiten Aspekt stellt die vorliegende Erfindung ein computerimplementiertes Verfahren zur dynamischen Optimierung des Kontrollpunkts mindestens eines robotisch ansteuerbaren Instruments bereit, zumindest umfassend die Schritte: fortlaufendes Erfassen von Informationen über eine Position und einen Kontrollpunkt mindestens eines robotisch ansteuerbaren Instruments; Ermitteln mindestens eines optimierten Kontrollpunkts auf Basis der Informationen über die Position und den Kontrollpunkt des mindestens einen robotisch ansteuerbaren Instruments; und Übertragen von Bewegungsbefehlen an einen Roboter, der das mindestens eine robotisch ansteuerbare Instrument steuert, wobei die Bewegungsbefehle dazu ausgelegt sind, den Roboter anzuweisen, das mindestens eine robotisch ansteuerbare Instrument von einem des mindestens einen optimierten Kontrollpunkts zu kontrollieren.

Das computerimplementierte Verfahren des zweiten Aspekts kann vorzugsweise mit dem System des ersten Aspekts ausgeführt werden. In ähnlicher Weise können die verschiedenen Ausführungsformen des Systems gemäß Ausführungsformen des computerimplementierten Verfahrens des zweiten Aspekts bedient werden. Das Verfahren kann somit gemäß allen in Bezug auf das erfindungsgemäße System beschriebenen Optionen, Varianten, Ausführungsformen und Verfeinerungen angepasst werden und umgekehrt.

Gemäß einem dritten Aspekt stellt die vorliegende Erfindung ein Master-Slave-System bereit, umfassend zumindest: ein robotisch ansteuerbares Instrument; einen Roboter, der so ausgelegt ist, das robotisch ansteuerbare Instrument von einem Kontrollpunkt aus zu kontrollieren; und ein System nach dem ersten Aspekt der Erfindung, wobei das System dazu eingerichtet ist, mindestens einen optimierten Kontrollpunkt zu ermitteln und Bewegungsbefehle an den Roboter zu übertragen, wobei die Bewegungsbefehle dazu ausgelegt sind, den Roboter anzuweisen, das zumindest eine robotisch ansteuerbare Instrument von einem des mindestens einen optimierten Kontrollpunkts aus zu kontrollieren.

Gemäß einem vierten Aspekt stellt die Erfindung ein Computerprogrammprodukt bereit, das ausführbaren Programmcode umfasst, welcher dazu eingerichtet ist, wenn er von einer Recheneinrichtung ausgeführt wird, das Verfahren gemäß einer Ausführungsform des zweiten Aspekts der vorliegenden Erfindung durchzuführen.

Gemäß einem fünften Aspekt stellt die Erfindung ein nicht-flüchtiges, computerlesbares Datenspeichermedium bereit, das ausführbaren Programmcode umfasst, welcher dazu eingerichtet ist, wenn er von einer Recheneinrichtung ausgeführt wird, das Verfahren gemäß einer Ausführungsform des zweiten Aspekts der vorliegenden Erfindung durchzuführen.

Das nicht-flüchtige, computerlesbare Datenspeichermedium kann jede Art von Computerspeicher, insbesondere Halbleiterspeicher, wie z.B. einen Festkörperspeicher, umfassen oder aus einem solchen bestehen. Das Datenträger kann auch eine CD, eine DVD, eine Blu-Ray-Disc, einen USB-Speicherstick oder dergleichen umfassen oder daraus bestehen.

Gemäß einem sechsten Aspekt stellt die Erfindung einen Datenstrom bereit, der ausführbaren Programmcode umfasst oder zum Erzeugen von ausführbarem Programmcode konfiguriert ist, welcher dazu eingerichtet ist, wenn er von einer Recheneinrichtung ausgeführt wird, das Verfahren gemäß einer Ausführungsform des zweiten Aspekts der vorliegenden Erfindung durchzuführen.

Weitere vorteilhafte Varianten, Optionen, Ausführungsformen und Modifikationen ergeben sich aus den folgenden Figuren, der ausführlichen Beschreibung, sowie aus den Ansprüchen. Es versteht sich jedoch, dass die detaillierte Beschreibung und die spezifischen Beispiele zwar bevorzugte Ausführungsformen der Erfindung angeben, aber nur zur Veranschaulichung angeführt werden, da verschiedene Änderungen und Modifikationen innerhalb des Umfangs der Erfindung für den Fachmann ersichtlich sind.

### Kurzbeschreibung der Figuren

Einzelne Ausführungsformen der vorliegenden Offenbarung werden anhand der folgenden Abbildungen im Detail erläutert werden. Die Bestandteile in den Zeichnungen sind nicht notwendigerweise maßstabsgetreu, sondern dienen dazu, die Grundsätze der vorliegenden Erfindung zu veranschaulichen. Teile in den verschiedenen Abbildungen, die den gleichen Elementen oder Verfahrensschritten entsprechen, wurden in den Abbildungen mit den gleichen Bezugszeichen versehen. Die Nummerierung von Verfahrensschritten dient zunächst nur zu deren Unterscheidung und impliziert nicht zwingend eine entsprechende Reihenfolge, wobei es jedoch eine Variante darstellt, die Schritte in der Reihenfolge ihrer Nummerierung durchzuführen. Mehrere Schritte können auch überlappend oder gleichzeitig durchgeführt werden. Von den Figuren zeigen:
- Fig. 1: ein schematisches Blockdiagramm zum Erläutern eines Systems gemäß einer Ausführungsform des ersten Aspekts der vorliegenden Erfindung;
- Fig. 2: eine schematische Darstellung von möglichen ermittelten Kontrollpunkten und Kontrollbereichen unter Benutzung des Systems nach dem ersten Aspekt der vorliegenden Erfindung;
- Fig. 3: eine schematische Darstellung, die einen möglichen Anwendungsfall für eine medizinische Prozedur gemäß einer Ausführungsform der vorliegenden Erfindung erläutert;
- Fig. 4: eine schematische Darstellung, die einen weiteren möglichen Anwendungsfall für eine medizinische Prozedur gemäß einer Ausführungsform der vorliegenden Erfindung erläutert;
- Fig. 5: ein schematisches Blockdiagramm zur Erläuterung eines Master-Slave-Systems gemäß einer Ausführungsform des dritten Aspekts der vorliegenden Erfindung;
- Fig. 6: ein schematisches Flussdiagramm zur Erläuterung eines computerimplementierten Verfahrens gemäß einer Ausführungsform des zweiten Aspekts der vorliegenden Erfindung;
- Fig. 7: ein schematisches Blockdiagramm eines Computerprogrammprodukts gemäß einer Ausführungsform des vierten Aspekts der vorliegenden Erfindung; und
- Fig. 8: ein schematisches Blockdiagramm eines nicht-flüchtigen computerlesbaren Datenspeichermediums gemäß einer Ausführungsform des fünften Aspekts der vorliegenden Erfindung.

### Detaillierte Beschreibung der Figuren

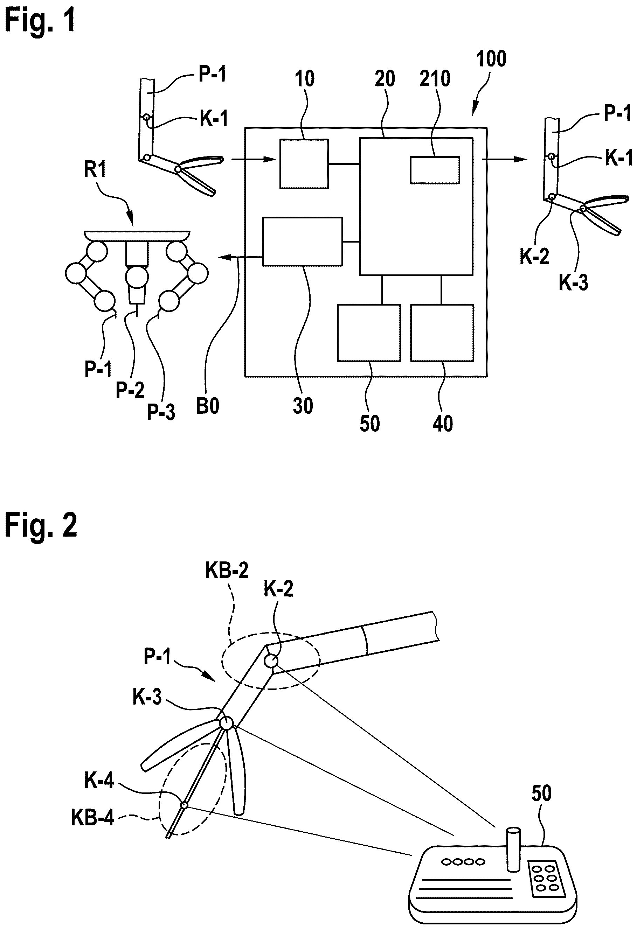

Fig. 1 zeigt ein schematisches Blockdiagramm zum Erläutern eines Systems 100 zur dynamischen Optimierung eines Kontrollpunkts K-1, K-2, K-3 (im Folgenden teilweise zusammenfassend mit K-i bezeichnet) mindestens eines robotisch ansteuerbaren Instruments P-1. Das in Fig. 1 dargestellte erfindungsgemäße System 100 umfasst eine Erfassungseinrichtung 10, eine Ermittlungseinrichtung 20, eine Steuerungseinrichtung 30, eine Datenbank 40 und eine Benutzerschnittstelle 50.

Die Erfassungseinrichtung 10 ist dazu eingerichtet, Informationen über eine Position und einen Kontrollpunkt des mindestens einen robotisch ansteuerbaren Instruments P-1 fortlaufend zu erfassen. In Fig. 1 ist als Beispiel für ein robotisch ansteuerbares Instrument P-1 eine Fasszange dargestellt, die bei einer minimalinvasiven endoskopischen Prozedur verwendet werden kann. Diese Fasszange wird von einem Roboter R1 gesteuert, wobei der Roboter R1 einen oder mehrere mehrgelenkige Roboterarme besitzen kann.

In der in Fig. 1 beispielhaft gezeigten Ausführungsform besitzt der Roboter R1 beispielsweise drei Mehrgelenkroboterarme. An seinem distalen Ende hat jeder Mehrgelenkroboterarm einen Instrumentenhalter, welcher die robotisch ansteuerbaren Instrumente P-1 bis P-3 (im Folgenden teilweise zusammenfassend als P-i bezeichnet) halten kann. Das robotisch ansteuerbare Instrument P-1 wird beispielsweise an einem Kontrollpunkt K-1 von dem Roboter R1 gehalten, der sich an einem Gelenk des robotisch ansteuerbaren Instruments P-1 befindet. Weitere Beispiele für ein robotisch ansteuerbares Instrument P-i können eine Zange, ein Nadelhalter oder ein Klammernahtgerät sein.

Die Erfassungseinrichtung 10 kann dazu eingerichtet sein, die Position und den Kontrollpunkt K-1 (d.h. den bestehenden oder aktuellen Kontrollpunkt K-1) des Instruments P-1 durch die Verarbeitung (vorzugsweise durch eine Bildverarbeitungssoftware) von Bilddaten einer oder mehrerer Bildgebungseinrichtungen (z.B. des Bildstroms eines Endoskops) zu erfassen, insbesondere in Echtzeit, oder alternativ auch von einem Bildablage- und Kommunikationssystem, wobei auch letzteres in Echtzeit erfolgen kann.

Die Ermittlungseinrichtung 20 ist dazu eingerichtet, auf Basis der Informationen über die Position und den bestehenden Kontrollpunkt K-1 des robotisch ansteuerbaren Instruments P-1, mindestens einen optimierten Kontrollpunkt K-i zu ermitteln. In Fig. 1 sind rechts beispielsweise ein optimierter Kontrollpunkt K-2 und ein optimierter Kontrollpunkt K-3 dargestellt. Es ist denkbar, dass der optimierte Kontrollpunkt K-2 zu einem Zeitpunkt ermittelt wird (mit dem robotisch ansteuerbaren Instrument P-1 in einer ersten Position und mit einem Kontrollpunkt K-1) und der optimierte Kontrollpunkt K-3 zu einem späteren Zeitpunkt ermittelt wird (mit dem robotisch ansteuerbaren Instrument P-1 in einer zweiten Position und mit einem Kontrollpunkt K-2). Es ist aber auch möglich, dass die Ermittlungseinrichtung 20 die Kontrollpunkte K-2 und K-3 gleichzeitig als optimierte Kontrollpunkte K-i anbietet. Die Kontrollpunkte K-2 und K-3 können dabei beispielsweise als gleichwertige Alternativen angeboten werden, oder gemäß einer vorbestimmten oder dynamisch berechneten Empfehlungsreihenfolge.

In einigen Ausführungsformen der Erfindung können die optimierten Kontrollpunkte K-i zu einer Liste von vorbestimmten Kontrollpunkten gehören. Diese Liste von Kontrollpunkten K-i kann in der Datenbank 40 gespeichert sein und von der Ermittlungseinrichtung 20 abgerufen werden. Bei diesen Ausführungsformen der Erfindung können die Rechenanforderungen der Ermittlungseinrichtung 20 relativ gering gehalten werden. Bei der in Fig. 1 dargestellten Ausführungsform der Erfindung könnten die Kontrollpunkte K-i die vorbestimmten Kontrollpunkte K-i für eine Fasszange sein. Die von der Ermittlungseinrichtung 20 ermittelten oder ermittelbaren Kontrollpunkte K-i beschränken sich dann auf die Kontrollpunkte K-1 bis K-3. Beispielsweise kann die Ermittlungseinrichtung 20 im Laufe einer medizinischen Prozedur die Abfolge von optimierten Kontrollpunkten K1-K3-K2-K1 ermitteln.

In einigen Ausführungsformen der Erfindung ist die Ermittlungseinrichtung 20 ferner dazu eingerichtet, einen optimierten Kontrollbereich KB-i zu ermitteln. In Fig. 2 sind beispielsweise drei optimierte Kontrollpunkte K-2, K-3 und K-4 zu erkennen. Abhängig davon kann das robotisch ansteuerbare Instrument P-1 aus Fig. 1 von einem Roboter R1 über das Gelenk, das Maul oder die Spitze als Kontrollpunkt K-i gesteuert werden. Der optimierte Kontrollpunkt K-2 befindet sich innerhalb eines optimierten Kontrollbereichs KB-2. Dementsprechend kann ein Operateur einen Kontrollpunkt K-i innerhalb des optimierten Kontrollbereichs KB-2 auswählen, um einen Kontrollpunkt zu finden, der seinen Gewohnheiten am besten entspricht. Zum Beispiel kann ein Bediener es vorziehen, ein Gelenk in der Mitte, von hinten oder von vorne zu halten, d.h. einen Kontrollpunkt an, distal zu, oder proximal zu einem Gelenk auszuwählen. Das gleiche gilt für den optimierten Kontrollpunkt KB-4. Ein Operateur kann einen Kontrollpunkt K-i innerhalb des Kontrollbereichs KB-4 auswählen, einschließlich des Kontrollpunkts K-4.

Die Ermittlungseinrichtung 20 kann jede Vorrichtung oder jedes Mittel zum Rechnen sein, insbesondere zum Ausführen einer Software, einer App oder eines Algorithmus. Die Ermittlungseinrichtung 20 kann eine künstliche-Intelligenz-Entität, KIE, 210 umfassen, die mit Modellen des maschinellen Lernens (oder Maschinenlernen-Modellen) ausgestattet ist. Die Algorithmen der Ermittlungseinrichtung 20 können daher zumindest teilweise mit Methoden der künstlichen Intelligenz unterstützt oder von diesen gespeist werden, oder umgekehrt. Diese Modelle sind dazu trainiert und ausgebildet, den(die) optimierten Kontrollpunkt(e) K-i automatisch auszuwählen.

Die KIE 210 kann den optimierten Kontrollpunkt K-i unter Berücksichtigung des jeweiligen robotisch ansteuerbaren Instruments P-1 und der Art der Prozedur bestimmen. Insbesondere kann der optimierte Kontrollpunkt K-i mithilfe einer Operationsplanung (OP-Planung) ermittelt werden, welche den Ablauf einer medizinischen Prozedur beschreibt. Die KIE 210 kann dabei beispielsweise mit Methoden der generativen künstlichen Intelligenz ausgestattet sein, so dass sie basierend auf den Anweisungen der OP-Planung die optimalen Kontrollpunkte K-i bestimmen kann. Eine solche OP-Planung kann beispielsweise von einer lokalen Benutzerschnittstelle oder von einer zentralen Recheneinrichtung (nicht in Fig. 1 dargestellt) von der Ermittlungseinrichtung 20 empfangen werden.

Die KIE 210 kann ferner dazu ausgelegt sein, die Kontrollpunkte K-i für jeden Operateur individuell, basierend auf vorgegebenen Präferenzen eines Operateurs anzupassen.

Die optimierten Kontrollpunkte K-i könnten auch mit Hilfe von Informationen mindestens eines optischen Sensors oder eines Kraftsensors bestimmt werden. Bilddaten eines optischen Sensors (z. B. der Kamera eines Endoskops) können verwendet werden, um die Position der Kontrollpunkte zu verfeinern. Bei diesen Ausführungsformen ist die künstliche-Intelligenz-Entität 210 vorzugsweise mit einer Bildverarbeitungssoftware ausgestattet, um eine Erkennung des Instruments P-i zu ermöglichen. Informationen über eine ausgeübte Kraft sind auch relevant, um einen besseren Kontrollpunkt zu finden, z.B. einen Kontrollpunkt K-i von dem aus die Kräfte effizienter übertragen oder besser kontrolliert werden können.

Die Steuerungseinrichtung 30 ist dazu eingerichtet, Bewegungsbefehle B0 an den Roboter R1 zu übertragen, wobei die Bewegungsbefehle B0 Informationen (vorzugsweise in Maschinensprache) enthalten, damit der Roboter R1 den Haltepunkt eines Instruments (in Bezug auf Fig. 1, von einem bestehenden Kontrollpunkt K-1 zu einem anderen Kontrollpunkt K-2 oder K-3) wechselt. Die Steuerungseinrichtung 30 kann mit Aktuatoren des Roboters R1 drahtverbunden oder drahtlos gekoppelt sein.

Die Benutzerschnittstelle 50 ist dazu ausgelegt, die optimierten Kontrollpunkte K-i anzuzeigen und einen von einem Benutzer (oder Operateur) ausgewählten Kontrollpunkt K-i als den optimierten Kontrollpunkt K-i an die Steuerungseinrichtung 30 zu übertragen. Die Benutzerschnittstelle 50 kann vorzugsweise dazu ausgelegt sein, den aktuellen (bestehenden) Kontrollpunkt K-1 zusammen mit dem mindestens einen ermittelten Kontrollpunkt K-2 und K-3 anzuzeigen.

Das in Fig. 1 dargestellte System 100 umfasst ferner eine Datenbank 40. In der Datenbank 40 können die ermittelten Kontrollpunkte K-i für einen Operateur als persönliches Konfigurationsprofil in einer Datei gespeichert sein. Auf diese Weise kann die Ermittlungseinrichtung 20 die von einem Operateur bevorzugten Kontrollpunkte K-i abrufen und dementsprechend die zu ermittelnden Kontrollpunkte K-i unter Berücksichtigung der Präferenzen des Chirurgen bestimmen. In der Datenbank 40 kann auch eine Liste mit vorbestimmten Kontrollpunkten K-i eines Instruments P-i gespeichert sein.

Das erfindungsgemäße System 100 ermöglicht es somit, den Kontrollpunkt K-i eines robotergesteuerten Instruments P-i während einer Prozedur dynamisch (d.h. fortlaufend oder kontinuierlich) automatisch anzupassen. Dadurch werden die Bewegungen eines Roboters R1 in Master-Slave-Systemen denen eines Chirurgen angenähert.

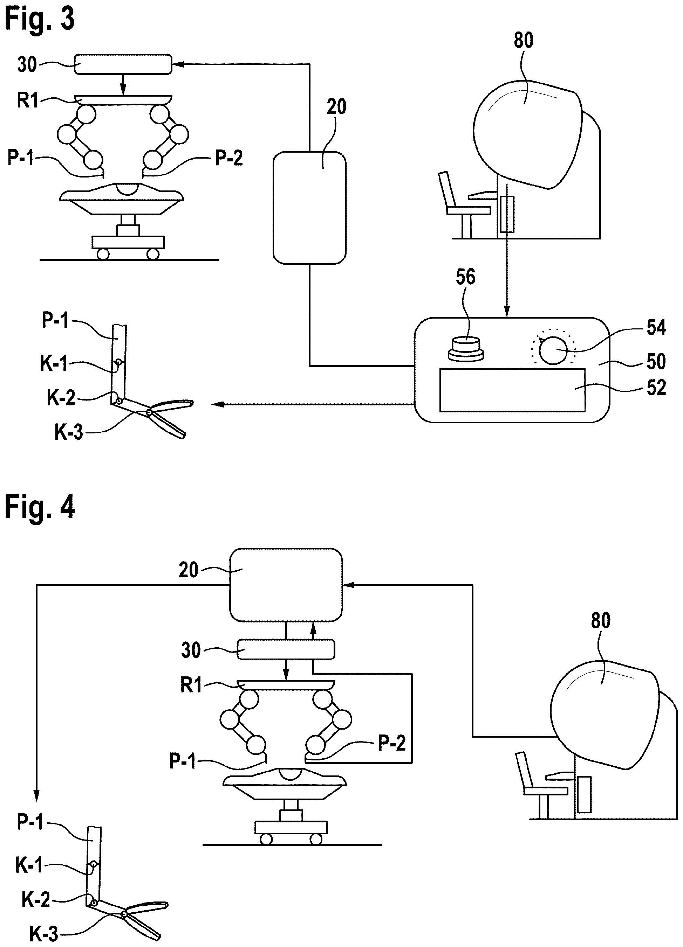

Fig. 3 zeigt eine schematische Darstellung, die einen möglichen Anwendungsfall für eine medizinische Prozedur gemäß einer Ausführungsform der vorliegenden Erfindung erläutert.

In Fig. 3 ist eine Benutzerschnittstelle 50 zu sehen, die von einem Operateur betätigt werden kann. Der Operateur kann den Roboter R1 mit einer Konsole 80 steuern. In einigen Ausführungsformen kann die Benutzerschnittstelle 50 in die Konsole 80 integriert sein oder umgekehrt.

Die Benutzerschnittstelle 50 umfasst beispielsweise zumindest ein Display 52 und einen Regler 54. Bei medizinischen Prozeduren, bei denen eine Mehrzahl von Instrumenten P-i von einem Roboter R1 gesteuert werden, kann die Benutzerschnittstelle 50 auch einen Mechanismus enthalten, mithilfe dessen der Operateur zunächst ein Instrument P-i aus der Mehrzahl von Instrumenten P-i auswählen kann. In Fig. 3 umfasst die Benutzerschnittstelle 50 hierfür einen Knopf 56. Auf dem Display 52 kann beispielsweise die Mehrzahl von Instrumenten P-i in einem Menü angezeigt werden, aus dem mittels des Knopfs 56 eines ausgewählt wird.

Auf dem Display 54 werden dann die Kontrollpunkte K-i (vorzugsweise der aktuelle Kontrollpunkt K-i zusammen mit den von der Ermittlungseinrichtung 20 ermittelten Kontrollpunkten K-i) und/oder die ermittelten Kontrollbereiche KB-i für das ausgewählte Instrument P-i angezeigt, vorzugsweise einem Bild des Instruments P-i überlagert. Der Operateur kann dann etwa mit dem Regler 56 durch das Bild des Instruments P-i navigieren, beispielsweise mit einem Cursor, und aus der Mehrzahl von Kontrollpunkten K-i einen gewünschten Kontrollpunkt K-i auszuwählen.

Bei der in Fig. 3 gezeigten Ausführungsform der Erfindung ist die Auswahl des Kontrollpunkts K-i dem Operateur überlassen. Gemäß diesen Ausführungsformen ist auch vorgesehen, dass der Operateur mit dem Regler einen gewünschten Kontrollpunkt K-i auswählt, der sich nicht unter den von der Ermittlungseinrichtung 20 ermittelten Kontrollpunkten K-i befindet. Die Auswahl wird dann an die Steuerungseinrichtung 30 übertragen, die entsprechende Bewegungsbefehle zur Steuerung des Roboters R1 erzeugt, um den Kontrollpunkt K-i entsprechend der Auswahl des Operateurs zu ändern.

Fig. 4 zeigt eine schematische Darstellung, die einen weiteren möglichen Anwendungsfall für eine medizinische Prozedur gemäß einer Ausführungsform der vorliegenden Erfindung erläutert.

Die in Fig. 4 dargestellte Ausführungsform der Erfindung stellt eine Alternative zu Fig. 3 dar. In der Variante gemäß Fig. 4 erfolgt die Auswahl des Kontrollpunkts K-i automatisch durch das System 100. Bei dieser Ausführungsformen ist die Ermittlungseinrichtung 20 dazu eingerichtet, die Ermittlung sowie die Auswahl des Kontrollpunkts K-i durchzuführen. Die Ermittlungseinrichtung 20, insbesondere die KIE 210, erfasst fortlaufend die Position des Instruments P-i und ermittelt zusammen mit dem aktuellen Kontrollpunkt K-i mindestens einen optimalen Kontrollpunkt K-i. Aus dem mindestens einen optimalen Kontrollpunkt K-i wird automatisch ein Kontrollpunkt K-i ausgewählt. Die Auswahl wird von der Steuerungseinrichtung 30 zur Erzeugung von Bewegungsbefehlen verwendet, sodass der Roboter das Instrument P-1 von dem ausgewählten Kontrollpunkt K-i aus kontrolliert.

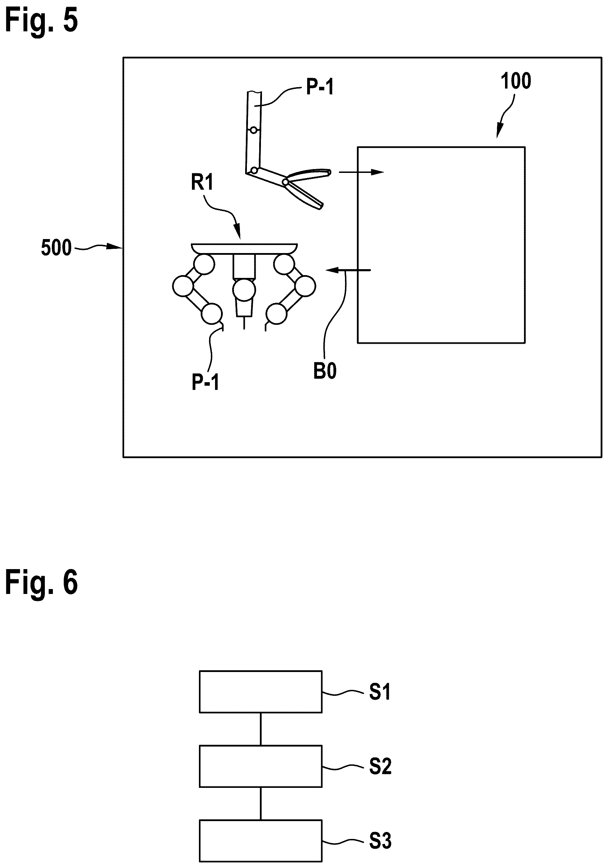

Fig. 5 zeigt ein schematisches Blockdiagramm zur Erläuterung eines Master-Slave-Systems 5ßß gemäß einer Ausführungsform des dritten Aspekts der vorliegenden Erfindung. Das Master-Slave-System 500 umfasst mindestens ein robotisch ansteuerbares Instrument P-1; einen Roboter R1, der dazu eingerichtet ist, das mindestens eine robotisch ansteuerbare Instrument P-1 von einem Kontrollpunkt K-i aus zu kontrollieren; und ein System 100 nach dem ersten Aspekt der Erfindung.

Das System 100 ist dazu eingerichtet, mindestens einen optimierten Kontrollpunkt K-i zu ermitteln und entsprechende Bewegungsbefehle B0 an den Roboter R1 zu übertragen, wobei die Bewegungsbefehle B0 dazu ausgelegt sind, den Roboter R1 anzuweisen, das zumindest eine robotisch ansteuerbare Instrument P-1 von einem des mindestens einen optimierten Kontrollpunkts K-i zu kontrollieren.

Fig. 6 zeigt ein schematisches Flussdiagramm zum Erläutern eines Verfahrens gemäß einer Ausführungsform des zweiten Aspekts der vorliegenden Erfindung, d.h., eines computerimplementierten Verfahrens zur dynamischen Optimierung des Kontrollpunkts K-i zumindest eines robotisch ansteuerbaren Instruments P-i. Das Verfahren gemäß Fig. 6 ist insbesondere mittels des Systems 100 aus Fig. 1 durchführbar und kann daher gemäß allen in Bezug auf das erfindungsgemäße System 100 beschriebenen Optionen oder Varianten angepasst werden und umgekehrt.

In einem Schritt S1 werden Informationen über eine Position und einen Kontrollpunkt K-i mindestens eines robotisch ansteuerbaren Instruments P-i fortlaufend erfasst, etwa wie im Vorangehenden in Bezug auf die Erfassungseinrichtung 10 beschrieben wurde.

In einem Schritt S2 wird mindestens ein optimierter Kontrollpunkts K-i auf Basis der Informationen über die Position und den Kontrollpunkt K-i des mindestens einen robotisch ansteuerbaren Instruments P-i ermittelt, etwa wie im Vorangehenden in Bezug auf die Ermittlungseinrichtung 20 beschrieben wurde.

In einem weiteren Schritt S3 werden Bewegungsbefehle B0 an einen Roboter R1 übertragen, der das mindestens eine robotisch ansteuerbare Instrument P-i steuert. Die Bewegungsbefehle B0 sind dazu ausgelegt, den Roboter R1 anzuweisen, das mindestens eine robotisch ansteuerbare Instrument P-i von einem des mindestens einen optimierten Kontrollpunkts K-i aus zu kontrollieren, etwa wie im Vorangehenden in Bezug auf die Steuerungseinrichtung 20 beschrieben wurde.

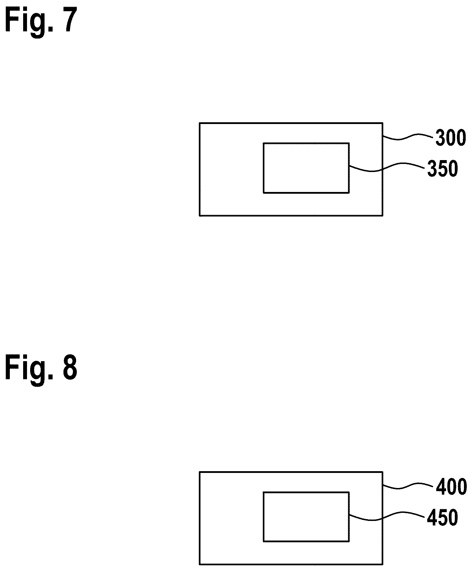

Fig. 7 zeigt ein schematisches Blockdiagramm eines Computerprogrammprodukts 300 gemäß einer Ausführungsform des dritten Aspekts der vorliegenden Erfindung. Das Computerprogrammprodukt 300 umfasst ausführbaren Programmcode 350, welcher dazu eingerichtet ist, wenn er ausgeführt wird (z.B. durch eine Recheneinrichtung), das Verfahren gemäß einer Ausführungsform der vorliegenden Erfindung durchzuführen, beispielsweise gemäß Fig. 6.

Fig. 8 zeigt ein schematisches Blockdiagramm eines nicht-flüchtigen computerlesbaren Datenspeichermediums 400 gemäß einer Ausführungsform der vorliegenden Erfindung. Das Datenspeichermedium 400 umfasst ausführbaren Programmcode 450, welcher dazu eingerichtet ist, wenn er ausgeführt wird (z.B. durch eine Recheneinrichtung), das Verfahren gemäß einer Ausführungsform des zweiten Aspekts der vorliegenden Erfindung durchzuführen, beispielsweise gemäß Fig. 6.

Das nicht-flüchtige computerlesbare Datenspeichermedium 400 kann beispielsweise als ein Halbleiterspeicher, z.B. ein SSD-Speicherstein ausgebildet sein oder einen solchen aufweisen. Das Datenspeichermedium 400 kann auch eine CD, DVD, Blu-Ray oder eine magnetische Speichervorrichtung aufweisen oder umfassen.

Die vorstehende Beschreibung der offenbarten Ausführungsformen enthält lediglich Beispiele für mögliche Umsetzungen, die beschrieben werden, um einen Fachmann in die Lage zu versetzen, die vorliegende Erfindung herzustellen oder verwenden zu können. Verschiedene Variationen und Modifikationen dieser Ausführungsformen sind für den Fachmann - nach Kenntnis der vorliegenden Erfindung - leicht ersichtlich, und die hierin definierten allgemeinen Prinzipien können auf andere Ausführungsformen angewendet werden, ohne vom Umfang der vorliegenden Offenbarung abzuweichen.

Somit soll die vorliegende Erfindung nicht auf die hierin gezeigten, spezifischen Ausführungsformen beschränkt sein, sondern ihr soll der breiteste Umfang zugestanden werden, der mit den hierin offenbarten Prinzipien und Merkmalen übereinstimmt.

### Bezugszeichenliste

- 10: Erfassungseinrichtung
- 20: Ermittlungseinrichtung
- 30: Steuerungseinrichtung
- 40: Datenbank
- 50: Benutzerschnittstelle
- 52: Display
- 54: Regler
- 56: Knopf
- 80: Konsole
- 100: System
- 210: Künstliche-Intelligenz-Entität
- 300: Computerprogrammprodukt
- 350: Programmcode
- 400: Datenspeichermedium
- 450: Programmcode
- 500: Master-Slave-System
- B0: Bewegungsbefehle
- P-i: robotisch ansteuerbares Instrument
- R1: Roboter
- K-i: Kontrollpunkte
- KB-i: Kontrollbereiche
- S1...S3: Verfahrensschritte

## Patentansprüche

1. System (100) zur dynamischen Optimierung eines Kontrollpunkts (K-i) mindestens eines robotisch ansteuerbaren Instruments (P-i), umfassend:
eine Erfassungseinrichtung (10), welche dazu eingerichtet ist, Informationen über eine Position und einen Kontrollpunkt (K-i) des mindestens einen robotisch ansteuerbaren Instruments (P-i) fortlaufend zu erfassen;
eine Ermittlungseinrichtung (20), welche dazu eingerichtet ist, auf Basis der Informationen über die Position und den Kontrollpunkt (K-i) des mindestens einen robotisch ansteuerbaren Instruments (P-i), mindestens einen optimierten Kontrollpunkt (K-i) zu ermitteln; und
eine Steuerungseinrichtung (30), welche dazu eingerichtet ist, Bewegungsbefehle (B0) an einen Roboter (R1), der das mindestens eine robotisch ansteuerbare Instrument (P-i) steuert, zu übertragen, wobei die Bewegungsbefehle (B0) dazu ausgelegt sind, den Roboter (R1) anzuweisen, das mindestens eine robotisch ansteuerbare Instrument (P-i) von einem des mindestens einen optimierten Kontrollpunkts (K-i) aus zu kontrollieren.

2. System (100) nach Anspruch 1,
wobei das robotisch ansteuerbare Instrument (P-i) ein medizinisches Instrument ist, insbesondere zur Anwendung bei minimalinvasiven endoskopischen Prozeduren.

3. System (100) nach einem der Ansprüche 1 oder 2,
wobei die Ermittlungseinrichtung (30) ferner dazu eingerichtet ist, einen optimierten Kontrollbereich (KB-i) zu ermitteln.

4. System (100) nach einem der Ansprüche 1 bis 3,
ferner umfassend eine Benutzerschnittstelle (50), die dazu ausgelegt ist, eine Mehrzahl von Kontrollpunkten (K-i) anzuzeigen und einen von einem Benutzer ausgewählten Kontrollpunkt (K-i) aus der Mehrzahl von Kontrollpunkten (K-i) als den optimierten Kontrollpunkt (K-i) an die Steuerungseinrichtung (30) zu übertragen.

5. System (100) nach Anspruch 4,
wobei die Benutzerschnittstelle (50) ein Display (52) und einen Regler (54) umfasst, wobei das Display (52) dazu ausgelegt ist, eine Mehrzahl von Kontrollpunkten (K-i) anzuzeigen, und wobei der Regler (54) so ausgelegt ist, dass ein Benutzer damit einen gewünschten Kontrollpunkt (K-i) aus der Mehrzahl von Kontrollpunkten (K-i) auswählen kann.

6. System (100) nach einem der Ansprüche 1 bis 5,
wobei die Ermittlungseinrichtung (20) ferner dazu eingerichtet ist, den optimierten Kontrollpunkt (K-i) aus einer Liste von vorbestimmten Kontrollpunkten (K-i) auszuwählen, wobei die Liste von vorbestimmten Kontrollpunkten (K-i) abhängig von dem robotisch ansteuerbaren Instrument (P-i) ist.

7. System (100) nach einem der Ansprüche 1 bis 6,
wobei die Ermittlungseinrichtung (20) ferner eine künstliche-Intelligenz-Entität (210) umfasst, die mit einem oder mehreren Maschinenlernen-Modellen ausgestattet ist, und die dazu trainiert und ausgebildet ist, einen optimierten Kontrollpunkt (K-i) automatisch auszuwählen.

8. System (100) nach Anspruch 7,
wobei die künstliche-Intelligenz-Entität (210) derart ausgelegt ist, den optimierten Kontrollpunkt (K-i) basierend auf Informationen über mindestens ein zu verwendendes robotisch ansteuerbares Instrument (P-i) und/oder über einen Benutzer und/oder über eine durchzuführende Prozedur automatisch auszuwählen.

9. System (100) nach einem der Ansprüche 7 oder 8,
wobei die künstliche-Intelligenz-Entität (210) derart ausgelegt ist, den optimierten Kontrollpunkt (K-i) mit Hilfe von Informationen mindestens eines optischen Sensors zu bestimmen, und
wobei der mindestens eine optische Sensor dazu eingerichtet ist, Bilder von zumindest dem robotisch ansteuerbaren Instrument (P-i) und dem Kontrollpunkt (K-i) zu erfassen.

10. System (100) nach einem der Ansprüche 7 bis 9,
wobei die künstliche-Intelligenz-Entität (210) ferner dazu ausgebildet ist, den optimierten Kontrollpunkt (K-i) gemäß einer Operationsplanung zu ermitteln.

11. System (100) nach einem der Ansprüche 1 bis 10,
ferner umfassend mindestens einen Kraftsensor, der dazu ausgelegt ist, Informationen über die von dem robotisch ansteuerbaren Instrument (P-i) ausgeübte Kraft zu erfassen und die erfassten Informationen an die Ermittlungseinrichtung (20) zu übertragen.

12. Computerimplementiertes Verfahren zur dynamischen Optimierung des Kontrollpunkts (K-i) zumindest eines robotisch ansteuerbaren Instruments (P-i), zumindest umfassend die Schritte:
fortlaufendes Erfassen (S1) von Informationen über eine Position und einen Kontrollpunkt (K-i) mindestens eines robotisch ansteuerbaren Instruments (P-i);
Ermitteln (S2) mindestens eines optimierten Kontrollpunkts (K-i) auf Basis der Informationen über die Position und den Kontrollpunkt (K-i) des mindestens einen robotisch ansteuerbaren Instruments (P-i); und
Übertragen (S3) von Bewegungsbefehlen (B0) an einen Roboter (R1), der das mindestens eine robotisch ansteuerbare Instrument (P-i) steuert, wobei die Bewegungsbefehle (B0) dazu ausgelegt sind, den Roboter (R1) anzuweisen, das mindestens eine robotisch ansteuerbare Instrument (P-i) von einem des mindestens einen optimierten Kontrollpunkts (K-i) aus zu kontrollieren.

13. Master-Slave-System (500) zur Durchführung medizinischer Prozeduren, zumindest umfassend:
ein robotisch ansteuerbares Instrument (P-i);
einen Roboter (R1), der dazu ausgelegt ist, das robotisch ansteuerbare Instrument (P-i) von einem Kontrollpunkt (K-i) aus zu kontrollieren; und
ein System (100) nach einem der Ansprüche 1 bis 11,
wobei das System (100) dazu eingerichtet ist, mindestens einen optimierten Kontrollpunkt (K-i) zu ermitteln und Bewegungsbefehle (B0) an den Roboter (R1) zu übertragen, wobei die Bewegungsbefehle (B0) dazu ausgelegt sind, den Roboter (R1) anzuweisen, das zumindest eine robotisch ansteuerbare Instrument (P-i) von einem des mindestens einen optimierten Kontrollpunkts (K-i) aus zu kontrollieren.

14. Computerprogrammprodukt (300),
umfassend ausführbaren Programmcode (350), welcher dazu eingerichtet ist, wenn er ausgeführt wird, das Verfahren gemäß Anspruch 12 durchzuführen.

15. Nicht-flüchtiges, computerlesbares Datenspeichermedium (400),
umfassend ausführbaren Programmcode (450), welcher dazu eingerichtet ist, wenn er ausgeführt wird, das Verfahren gemäß Anspruch 12 durchzuführen.
